# EUROPEAN PATENT APPLICATION

(11) **EP 1 923 697 A1**
(43) Date of publication of application: **21.05.2008**
(21) Application number: 07022134.6
(22) Date of filing: 14.11.2007
(51) Int. Cl.: G01N 27/12

(54) **High sensitive resistive-type gas sensor and its manufacturing process comprising an organic-inorganic intercalated hydbrid sensing material**

(30) Priority: 18.11.2006 JP 2006312309
(71) Applicant: National Institute of Advanced Industrial Science and Technology, Tokyo 100-8921 (JP)
(72) Inventor: Itoh, Toshio, Moriyami-ku, Nagoya-shi, Aichi 463-8560 (JP); Matsubara, Ichiro, Moriyami-ku, Nagoya-shi, Aichi 463-8560 (JP); Shin, Woosuck, Moriyami-ku, Nagoya-shi, Aichi 463-8560 (JP); Izu, Noriya, Moriyami-ku, Nagoya-shi, Aichi 463-8560 (JP)
(74) Representative: Ebner von Eschenbach, Jennifer

(57) **Abstract**

Gas sensor element for detecting an aldehyde gas in concentrations of several tens of ppb, a process for manufacturing such a material, and a gas sensor element and the like comprising such a material. The gas sensor material comprises an organic-inorganic hybrid material in which a conductive organic polymer is intercalated between layers of an inorganic compound having a layer structure and from which a conductive organic polymer not intercalated between the layers of the inorganic compound is removed, and provides a process for manufacturing such a gas sensor material, as well as a chemical sensor member thus providing a chemical sensor material by which the gas sensor material can detect by itself an aldehyde gas in concentrations of several tens of ppb without using a sensitivity-enhancing element such as a gas-concentrating element.

## Description

### BACKGROUND OF THE INVENTION

### 1. Field of the invention

The present invention relates to a high sensitive gas sensor and its manufacturing process. More particularly, the invention relates to: a gas sensor using as a chemical sensor element a specific organic-inorganic hybrid material, the gas sensor having excellent long-term stability and affording detection of a gas on the basis of changes in resistance values; a gas sensor material comprising a conductive organic-inorganic hybrid material having the characteristic of detecting an aldehyde gas in concentrations of several tens of ppb at a temperature not exceeding 100°C; its manufacturing process; and a chemical sensor member comprising an organic-inorganic hybrid manufactured in accordance with such a process.

In the technical field of gas sensors using high-performance organic-inorganic hybrid materials in which an organic compound and an inorganic compound are combined at the nano level, conventional organic-inorganic hybrid materials exhibited selective response towards aldehyde gases, which are a kind of volatile organic compounds (VOCs). However, these materials were problematic in that detection of ultra-low concentrations, of several tens of ppb, was difficult, and hence the present invention has been developed with the goal of solving this problem.

### 2. Description of the Related Art

In recent years, there has come to light the health problem of the so-called "sick building syndrome", which is caused on the one hand by high air-tightness of buildings and on the other by pollution from VOCs that are generated through the use of building materials, interior work and the like, that release organic substances. This has prompted the development of chemical sensors for monitoring VOC concentrations indoors. Although VOC components are very varied, the allowable concentrations thereof are of several tens of ppb for most of them. Concerning the sick building syndrome (indoor air pollution), a Commission of the Ministry of Health, Labor and Welfare stipulated in 2002 a concentration of 0.08 ppm (= 80 ppb) for formaldehyde, and 0.03 ppm (=30 ppb) for acetaldehyde, both of which are aldehyde gases, as individual concentration guideline values, which are values judged to exert no harmful influence on health after lifelong inhalation of the respective compound at the given concentrations.

Since VOCs influence thus health even at ultra-low concentrations, chemical sensors for constant VOC monitoring are therefore required to possess enough sensitivity so as to identify and detect ultra-low concentrations of VOCs. Moreover, what is required for achieving a small-size and inexpensive concentration detection device is that the chemical sensor itself be capable of detecting VOCs at ultra-low concentrations rather than resorting to a sensitivity-enhancing element such as a gas-concentrating element or the like.

Methods for detecting ultra-low VOC concentrations, among methods conventionally used for measuring VOC gas concentrations, include, for instance, analysis by gas chromatography. Gas chromatography allows separating the components of a gas and measuring accurately the concentration of the components, but does not permit instantaneous measurement, owing to the nature of the analysis equipment. Hence, gas chromatography devices are not suitable for constant monitoring of VOC concentrations, while the high cost of the analysis equipment itself is another obstacle for widespread adoption of gas chromatography in VOC sensors installed in houses or office buildings. Portable analyzers have also been developed in which ultra-low concentrations of VOCs can be detected by a detector itself, on the basis of hydrogen flame ionization and photoionization detection, but owing to the nature of the detectors, gas specificity is difficult to determine in these analyzers.

In chemical sensors based on organic-inorganic hybrid materials, an organic compound and an inorganic compound are combined at the nano level in such a way that the layer-like inorganic compound and the organic compound interposed between the layers of the former are imparted with a signal transduction function, required for applications in electronic device materials, and with a molecule recognition function, required for gas specificity, to afford thereby an organic-inorganic hybrid material effectively used as a novel chemical sensor material having high gas specificity. It has been found that, in chemical sensors based on organic-inorganic hybrid materials, different response characteristics to various gases are obtained by varying the interlayer organic compound in an already-existing hybrid material comprising an organic compound intercalated between layers of layer-structure molybdenum oxide (MoO₃) (Japanese Laid-Open Patent Publication No. 2005-321326 and Bull. Chem. Soc. Jpn., Vol. 77, 1231 (2004)). Herein, a technology for making such a material into a thin film (Japanese Laid-Open Patent Publication No. 2005-179115, Chem. Mater., Vol. 17, 349 (2005)), a technology for increasing sensitivity (Japanese Laid-Open Patent Publication No. 2005-321327), and a technology for manufacturing a thin film on a silicon substrate having coated thereon a buffer layer (Japanese Patent Application No. 2005-142706:Japanese Laid-Open Patent Publication No. 2006-315933) make it possible not only to detect 6 ppm of an aldehyde gas, but also to achieve a selective response towards aldehyde gases alone in concentrations of 50 ppm or less.

Organic-inorganic hybrid materials comprising molybdenum oxide and an organic compound hold promise as novel chemical sensor materials for aldehyde gases. Although the above organic-inorganic hybrid materials respond to aldehyde gases in concentrations from several ppm, they fail to detect ultra-low concentrations of several tens of ppb, which are also health-damaging concentrations. To overcome that shortcoming, a sensitivity enhancing element such as a gas-concentrating element might conceivably be used concomitantly, but from the viewpoint of achieving a small-size, inexpensive concentration detection device, it is still imperative to develop an organic-inorganic hybrid material that enables a chemical sensor element to identify and detect by itself ultra-low concentrations of VOCs.

### SUMMARY OF THE INVENTION

Under such circumstances, and as a result of diligent research directed at, in the light of the above conventional technologies, developing a chemical sensor for detecting a gas through changes in resistance values, on the basis of an organic-inorganic hybrid material that detects an aldehyde gas in concentrations of several tens of ppb, the present inventors found out that, in a process for manufacturing a high-performance organic-inorganic hybrid material through compound combination, a gas sensor element capable of detecting an aldehyde gas in concentrations of several tens of ppb could be achieved by carrying out compound combination after having removed from a solvent the organic polymer of an insoluble component, in a stage prior to compound combination, thereby perfecting, upon further research, the present invention.

An object of the present invention is to provide a process for manufacturing a conductive organic-inorganic hybrid material that allows manufacturing a chemical sensor based on the conductive organic-inorganic hybrid material, for detecting an aldehyde gas in concentrations of several tens of ppb. Another object of the present invention is to provide an article of the organic-inorganic hybrid material, in particular a gas sensor element, a conductive member, and a high sensitive chemical sensor member.

The present invention comprises the following technical aspects:
(1) A gas sensor material as a high sensitive gas sensor material for detecting an aldehyde gas in concentrations of several tens of ppb, comprising an organic-inorganic hybrid material in which a conductive organic polymer is intercalated between layers of an inorganic compound having a layer structure, in which the conductive organic polymer not intercalated between the layers of the inorganic compound is removed, and having sensitivity increased so as to detect an aldehyde gas in concentrations of several tens of ppb, on the basis of changes in resistance values.
(2) The gas sensor material according to (1), wherein the shape of the organic-inorganic hybrid material, in which a conductive organic polymer is intercalated between layers of an inorganic compound having a layer structure, and from which a conductive organic polymer not intercalated between the layers of the inorganic compound is removed, is that of an oriented film.
(3) The gas sensor material according to (1), wherein the inorganic compound having a layer structure is a compound having molybdenum oxide as a main component.
(4) The gas sensor material according to (1), wherein the organic conductive polymer is a polymer having polyaniline as a main component.
(5) The gas sensor material according to (1), wherein the organic conductive polymer is a polymer having a polyaniline derivative as a main component.
(6) The gas sensor material according to (5), having as a main component a polyaniline derivative having an alkoxy group at the ortho position of the benzene ring.
(7) A method for manufacturing a gas sensor material as a high sensitive gas sensor material for detecting an aldehyde gas in concentrations of several tens of ppb, comprising the steps of:
   1) intercalating a conductive organic polymer between layers of an inorganic compound having a layer structure by using an aqueous solution from which an insoluble conductive organic polymer is removed, in a process of intercalating the conductive organic polymer between the layers of the inorganic compound;
   2) producing thereby the gas sensor material having the organic-inorganic hybrid material in which the conductive organic polymer not intercalated between the layers of the inorganic compound is removed; and
   3) increasing thereby sensitivity thereof so as to detect an aldehyde gas in concentrations of several tens of ppb, on the basis of changes in resistance values.
(8) The process for manufacturing a gas sensor material according to (7), wherein the shape of the organic-inorganic hybrid material, in which a conductive organic polymer is intercalated between layers of an inorganic compound having a layer structure, and from which a conductive organic polymer not intercalated between the layers of the inorganic compound is removed, is that of an oriented film.
(9) A chemical sensor member, comprising the gas sensor material defined in any one of (1) to (6) as a sensor element, and having a characteristic of detecting an aldehyde gas in concentrations of several tens of ppb.

The present invention is explained in detail next.

The present invention is a high sensitive gas sensor material for detecting an aldehyde gas in concentrations of several tens of ppb, being a gas sensor material comprising an organic-inorganic hybrid material in which a conductive organic polymer is intercalated between layers of an inorganic compound having a layer structure; wherein a conductive organic polymer not intercalated between the layers of the inorganic compound is absent, through removal; and wherein sensitivity is increased until detection of an aldehyde gas in concentrations of several tens of ppb, on the basis of changes in resistance values. In a preferred embodiment of the present invention, the shape of the organic-inorganic hybrid material, in which a conductive organic polymer is intercalated between layers of an inorganic compound having a layer structure, and in which the conductive organic polymer not intercalated between the layers of the inorganic compound is removed, is that of an oriented film.

Also, the present invention is a process for manufacturing a high sensitive gas sensor material for detecting an aldehyde gas in concentrations of several tens of ppb, comprising the steps of: in a process of intercalating a conductive organic polymer between layers of an inorganic compound having a layer structure, intercalating the conductive organic polymer between the layers of the inorganic compound by using an aqueous solution from which an insoluble conductive organic polymer is removed; manufacturing thereby a gas sensor material comprising an organic-inorganic hybrid material having removed therefrom a conductive organic polymer not intercalated between the layers of the inorganic compound; and increasing sensitivity until detection of an aldehyde gas in concentrations of several tens of ppb, on the basis of changes in resistance values.

A distinctive characteristic of the present invention is a gas sensor material for detecting an aldehyde gas in concentrations of several tens of ppb through changes in the resistance value of the gas sensor material, by using, as the gas sensor material, an oriented film of an organic-inorganic hybrid material in which a conductive organic polymer is intercalated between layers of an inorganic compound having a layer structure.

Another distinctive characteristic of the present invention is carrying out compound combination after having removed an insoluble component organic polymer from a solvent, in a stage prior to compound combination in a process for manufacturing a high-performance organic-inorganic hybrid material through combination of an inorganic compound and an organic compound. The present invention has been developed based on the novel finding of the inventors to the effect that a gas sensor material capable of detecting an aldehyde gas in concentrations of several tens of ppb can be obtained by preventing bulk conductive organic polymer from contaminating the hybrid oriented film, the bulk conductive organic polymer exhibiting a response of decreasing resistance value towards aldehyde gases, which is the opposite response to that exhibited by an organic-inorganic hybrid material, i.e. of increasing resistance value towards aldehyde gases.

In the present invention, the organic-inorganic hybrid material, in which a conductive organic polymer is intercalated between layers of an inorganic compound having a layer structure, and from which a conductive organic polymer not intercalated between the layers of the inorganic compound is removed, is used as a molded article such as an oriented film or the like. In the present invention, molding the above inorganic compound into a film has the effect of contributing to enhancing adhesiveness so that, in the process of intercalating a conductive organic polymer between the layers of an inorganic compound, the inorganic compound does not detach from the substrate, and has also the effect of realizing the use of the inorganic compound as a gas sensor material, through molding into a film. In the present invention, the form of the film, i.e. the shape and structure of the above molded product, can be set arbitrarily.

A compound having molybdenum oxide as a main component is used as the inorganic compound having the above layer structure. This allows imparting a signal transduction function to the organic-inorganic hybrid material. Also, a polymer having as a main component polyaniline or a polyaniline derivative is used as the above conductive organic polymer. This allows imparting a molecule recognition function to the organic-inorganic hybrid material. Although not limited thereto, the polyaniline or the polyaniline derivative of the present invention has as a main component a polyaniline derivative having an alkoxy group at the ortho position of the benzene ring.

The invention allows also providing a process for manufacturing a gas sensor material for detecting an aldehyde gas in concentrations of several tens of ppb, on the basis of changes in resistance values using a gas sensor material comprising an organic-inorganic hybrid material in which a conductive organic polymer is intercalated between layers of an inorganic compound having a layer structure, the organic-inorganic hybrid material being free, through removal therefrom, of conductive organic polymer not intercalated between the layers of the inorganic compound. Manufacturing a gas sensor element for detecting an aldehyde gas in concentrations of several tens of ppb, by means of an organic-inorganic hybrid material, is made possible, in the manufacturing process of obtaining a high-performance organic-inorganic hybrid material through combination of an organic polymer and an inorganic compound by intercalating the organic polymer between layers of the inorganic compound, by carrying out such combination after having removed from a solvent the organic polymer of an insoluble component using separation means such as filtration or the like, in a stage prior to compound combination.

The present invention affords a process for manufacturing an oriented film using an organic-inorganic hybrid material in which a conductive organic polymer is intercalated between layers of an inorganic compound having a layer structure, the organic-inorganic hybrid material having removed therefrom conductive organic polymer not intercalated between the layers of the inorganic compound. This allows manufacturing, and increasing the performance of, a gas sensor material for detecting an aldehyde gas in concentrations of several tens of ppb by means of an organic-inorganic hybrid material provided, as an oriented film, on substrate.

The present invention affords a chemical sensor member having as a constituent element thereof a gas sensor element comprising the above organic-inorganic hybrid material. The chemical sensor element can be imparted herein with the ability of detecting gas components by connecting the sensor material to an electrode and by monitoring the changes in resistance values.

As explained above, the present invention provides, for instance, a gas sensor element, and its manufacturing process, the gas sensor element comprising a conductive organic-inorganic hybrid material having the characteristic and function of detecting an aldehyde gas in concentrations of several tens of ppb, as a chemical sensor for detecting a gas based on changes in resistance values, the chemical sensor comprising herein an organic-inorganic hybrid material that is manufactured through a process of intercalating polyaniline, or a polyaniline derivative having an alkoxy group at the ortho position of the benzene ring, between layers of molybdenum oxide having a nano-size layer structure, out of an aqueous solution from which insoluble conductive organic polymer is removed by separation means such as filtration or the like, so that conductive organic polymer that is not intercalated between layers of the inorganic compound is removed from the organic-inorganic hybrid material.

An explanation follows next on the mechanism of a chemical sensor for measuring VOC concentrations using a gas sensor element comprising a conductive organic-inorganic hybrid material. The conductivity of the organic-inorganic hybrid material arises from charge transfer between the organic compound and the inorganic compound. In such an organic-inorganic hybrid material, intrusion of a VOC gas between layers of the organic-inorganic hybrid material causes a change in the charge transfer balance, owing to interactions between the VOC gas and the organic compound, which in turn gives rise to fluctuations in the electric resistance of the organic-inorganic hybrid material.

When exposed to a VOC gas, the conductive organic polymer itself exhibits electric resistance fluctuations. As a result, when the conductive organic-inorganic hybrid material is contaminated with conductive organic polymer that is not intercalated between layers, the electric resistance response of the conductive organic-inorganic hybrid material is hampered by the response of the conductive organic polymer that is not intercalated between layers. In terms of enhancing the sensitivity of a chemical sensor using the conductive organic-inorganic hybrid material, thus, there is preferably no contamination by conductive organic polymer that is not intercalated between layers.

In the present invention, the chemical sensor comprises a chemical sensor material for detecting an aldehyde gas in concentrations of several tens of ppb, preferably a conductive organic-inorganic hybrid material of molybdenum oxide and polyaniline, from which there is removed the polyaniline not intercalated between layers of the inorganic compound (such a substance is denoted hereafter as (PANI)ₓMoO₃), or a conductive organic-inorganic hybrid material of molybdenum oxide and poly(o-anisidine), from which there is removed the poly(o-anisidine) not intercalated between layers of the inorganic compound (such a substance is denoted hereafter as (PoANIS)ₓMoO₃).

Although conventional organic-inorganic hybrid materials having molybdenum oxide as a main component respond to an aldehyde gas in concentrations from several ppm upwards, they fail to detect ultra-low concentrations of several tens of ppb, which are health-damaging concentrations. By contrast, the (PANI)ₓMoO₃ and (PoANIS)ₓMoO₃ of the present invention, having removed therefrom conductive organic polymer that is not intercalated between layers of the inorganic compound, affords a chemical sensor material that detects an aldehyde gas in concentrations of several tens of ppb.

The resistance value of polyaniline or poly(o-anisidine), which are conductive polymers, decreases upon exposure to a polar aldehyde gas. This is caused by an increase in carriers resulting from the so-called electron donation that polyaniline undergoes when polar VOC molecules attach thereto. In (PANI)ₓMoO₃ or (PoANIS)ₓMoO₃, charge transfer takes place between the molybdenum oxide having a layer structure and the interlayer polyaniline or poly(o-anisidine). A change in the charge transfer balance gives rise to a change in the resistance value of (PANI)ₓMoO₃ or (PoANIS)ₓMoO₃.

Upon exposure to a polar aldehyde gas, the latter interacts with the interlayer polyaniline or poly(o-anisidine), thereby modifying the charge transfer balance, and causing the resistance value of (PANI)ₓMoO₃ or (PoANIS)ₓMoO₃ to rise. The resistance value of many hybrids of molybdenum oxide and organic compounds rises upon exposure to an aldehyde gas. On the other hand, the resistance value response of polyaniline and poly(o-anisidine) to aldehyde gases is just the reverse of that of (PANI)ₓMoO₃ or (PoANIS)ₓMoO₃. In order to achieve further enhanced sensitivity in (PANI)ₓMoO₃ or (PoANIS)ₓMoO₃, therefore, it is necessary to prevent contamination by polyaniline or poly(o-anisidine) that is not intercalated between layers of molybdenum oxide.

On the basis of that finding, contamination by polyaniline or poly(o-anisidine) that is not intercalated between layers of molybdenum oxide is prevented in the present invention by using a manufacturing process that employs an aqueous solution from which insoluble conductive organic polymer is removed by filtration, in the process of intercalating polyaniline or poly(o-anisidine) between layers of molybdenum oxide.

A hybrid of molybdenum oxide and polyaniline, virtually free of polyaniline or poly(o-anisidine) not intercalated between layers of molybdenum oxide, is synthesized through a two-stage reaction. [Na(H₂O)₂]ₓMoO₃, having hydrated sodium ions intercalated between molybdenum oxide layers, is synthesized in the first stage. Disodium molybdenate (VI) dihydrate and sodium hyposulfite are added to distilled water through which nitrogen gas is bubbled, then a molybdenum oxide thin film, formed beforehand on a substrate having provided thereon a gold comb electrode, is reacted with the resulting solution through dipping in the latter. The dipping time ranges preferably from 20 to 30 seconds. [Na(H₂O)₂]ₓMoO₃ is obtained after washing and drying.

In the subsequent second stage, polyaniline or poly(o-anisidine) is intercalated through ion exchange with the hydrated sodium ions (Na(H₂O)₂⁺) that are present between the [Na(H₂O)₂]ₓMoO₃ layers. Aniline or o-anisidine are dissolved in an aqueous solution of hydrochloric acid, to yield aniline hydrochloride or o-anisidine hydrochloride, followed by aniline polymerization through addition of an ammonium persulfate solution, as a polymerization initiator, to yield polyaniline or poly(o-anisidine). The polymerization time is preferably of about 30 minutes.

The insoluble polyaniline or poly(o-anisidine) is removed then using a hydrophilic PTFE membrane filter. The time devoted to this operation is preferably of about 80 minutes. Thereafter, the [Na(H₂O)₂]ₓMoO₃ is dipped in the obtained polyaniline aqueous solution or poly(o-anisidine) aqueous solution, to elicit an ion exchange reaction between the hydrated sodium ions and the dissolved polyaniline or poly(o-anisidine). The dipping time ranges preferably from about 20 to about 30 seconds. (PANI)ₓMoO₃ or (PoANIS)ₓMoO₃ is obtained after washing and drying.

An explanation follows next on the results of evaluations of the chemical sensor characteristic of the obtained (PANI)ₓMoO₃ or (PoANIS)ₓMoO₃. Resistance values were monitored using a comb electrode. Herein, changes in the resistance value, resulting from changes in the charge transfer balance in a VOC atmosphere of a given concentration, were taken as the sensor response. Although conventional organic-inorganic hybrid materials respond to aldehyde gases in concentrations from several ppm upward, they fail to detect ultra-low concentrations of several tens of ppb, which are also health-damaging concentrations. The chemical sensor based on (PANI)ₓMoO₃ or (PoANIS)ₓMoO₃ according to the present invention, however, detected an aldehyde gas in concentrations of several tens of ppb.

Conventional chemical sensors of polyaniline and molybdenum oxide that respond to aldehyde gases in concentrations from several ppm upwards have a stronger response to formaldehyde than to acetaldehyde. The (PANI)ₓMoO₃ of the present invention exhibits the same tendency at an ultra-low concentration region of several tens of ppb. Conventional chemical sensors of poly(o-anisidine) and molybdenum oxide that respond to aldehyde gases in concentrations from several ppm upwards have a stronger response to acetaldehyde than to formaldehyde. However, at an ultra-low concentration region of several tens of ppb, the (PoANIS)ₓMoO₃ of the present invention exhibits a difference in response to acetaldehyde and formaldehyde smaller than that of (PANI)xMoO₃, and hence (PoANIS)ₓMoO₃ exhibits thus a stronger response to acetaldehyde, and possesses higher specificity, than (PANI)ₓMoO₃.

In the present invention, the gas sensor material for detecting an aldehyde gas in concentrations of several tens of ppb based on the above organic-inorganic hybrid material is ideally used as a chemical sensor element for detecting gas components through monitoring of changes in the resistance value of the gas sensor material. Connecting this gas sensor material to a suitable electrode allows monitoring changes in resistance values and allows hence the gas sensor material to function as a chemical sensor element.

By combining an inorganic compound and an organic polymer after having removed an insoluble component organic polymer from a solvent, in a stage prior to combination of a inorganic compound and an organic compound in a process for manufacturing a high-performance organic-inorganic hybrid material through compound combination, the present invention allows providing a chemical sensor element, and its manufacturing process, such that the chemical sensor element comprises an organic-inorganic hybrid material having the characteristic of detecting an aldehyde gas in concentrations of several tens of ppb, which was not possible for a chemical sensor comprising a conventional organic-inorganic hybrid material.

The present invention provides a novel technology relating to a chemical sensor element comprising a conductive organic-inorganic hybrid material having, as main components, for instance polyaniline or poly(o-anisidine), as a conductive organic polymer, and molybdenum oxide, as an inorganic compound. Moreover, the present invention is useful in providing a novel technology relating to a gas sensor material that allows a chemical sensor comprising an organic-inorganic hybrid material to detect by itself an aldehyde gas in concentrations of several tens of ppb, without using a sensitivity-enhancing element such as a gas-concentrating element or the like.

The invention affords thus the following effects.
(1) The present invention provides a novel gas sensor material, comprising an organic-inorganic hybrid material, for detecting an aldehyde gas in concentrations of several tens of ppb.
(2) As a result, a chemical sensor comprising the organic-inorganic hybrid material can detect by itself an aldehyde gas in concentrations of several tens of ppb, without using a sensitivity-enhancing element such as a gas-concentrating element or the like.
(3) The present invention allows providing a small-size and inexpensive concentration detection device for detecting an aldehyde gas in concentrations of several tens of ppb.
(4) Using the detection device of the present invention allows constant monitoring of concentrations of an aldehyde gas in concentrations of several tens of ppb.
(5) The present invention allows providing a technology for controlling the response to various aldehyde gases in concentrations of several tens of ppb, on the basis of the interlayer organic compound in the gas sensor material comprising the organic-inorganic hybrid material.
(6) The present invention allows detecting concentrations up to the individual concentration guideline values stipulated in 2002 by the Ministry of Health, Labor and Welfare, in particular for formaldehyde.

### BRIEF DESCRIPTION OF THE DRAWINGS

Fig. 1 is a diagram illustrating an X-ray diffraction pattern of a MoO₃ thin film of Example 1;
Fig. 2 is a diagram illustrating an X-ray diffraction pattern of a [Na(H₂O)₂]ₓMoO₃ thin film of Example 1;
Fig. 3 is a diagram illustrating an X-ray diffraction pattern of a (PANI)ₓMoO₃ thin film of Example 1;
Fig. 4 is a schematic diagram of an apparatus for measuring electric characteristic and sensor characteristic in Example 1, illustrating a state in which clean nitrogen flows into a sample chamber;
Fig. 5 is a schematic diagram of an apparatus for measuring electric characteristic and sensor characteristic in Example 1, illustrating a state in which a gas to be measured flows, at a given concentration, into a sample chamber;
Fig. 6 is a diagram illustrating measurement results of sensor characteristic of a chemical sensor based on a (PANI)ₓMoO₃ thin film in Example 1, for 25 ppb and 400 ppb of formaldehyde;
Fig. 7 is a diagram illustrating measurement results of sensor characteristic of the chemical sensor based on the (PANI)ₓMoO₃ thin film of Example 1, for 50 ppb, 75 ppb, 100 ppb and 200 ppb of formaldehyde;
Fig. 8 is a diagram illustrating measurement results of sensor characteristic of the chemical sensor based on the (PANI)ₓMoO₃ thin film of Example 1, for 25 ppb, 50 ppb, 75 ppb, 100 ppb, 200 ppb and 400 ppb of acetaldehyde;
Fig. 9 is a diagram illustrating a blank measurement of a chemical sensor based on the (PANI)ₓMoO₃ thin film in Example 1;
Fig. 10 is a graph plotting the differences between response values for various concentrations of formaldehyde and the response values for blank measurement, in the chemical sensor based on the (PANI)ₓMoO₃ thin film of Example 1;
Fig. 11 is a graph plotting the differences between response values for various concentrations of acetaldehyde and the response values for blank measurement, in the chemical sensor based on the (PANI)ₓMoO₃ thin film of Example 1;
Fig. 12 is a diagram illustrating measurement results of sensor characteristic of a chemical sensor based on a (PANI)ₓMoO₃ thin film, for 10 ppm of acetaldehyde, in Example 2;
Fig. 13 is a scanning electron micrograph of the (PANI)ₓMoO₃ thin film of Example 2;
Fig. 14 is a diagram illustrating an X-ray diffraction pattern of a [Na(H₂O)₂]ₓMoO₃ thin film of Example 3;
Fig. 15 is a diagram illustrating an X-ray diffraction pattern of a (PoANIS)ₓMoO₃ thin film of Example 3;
Fig. 16 is a diagram illustrating measurement results of sensor characteristic of a chemical sensor based on the (PoANIS)ₓMoO₃ thin film of Example 3, for 25 ppb, 50 ppb, 75 ppb, 100 ppb, 200 ppb and 400 ppb of formaldehyde;
Fig. 17 is a diagram illustrating measurement results of sensor characteristic of the chemical sensor based on the (PoANIS)ₓMoO₃ thin film of Example 3, for 25 ppb, 50 ppb, 75 ppb, 100 ppb, 200 ppb and 400 ppb of acetaldehyde;
Fig. 18 is a diagram illustrating blank measurement of the chemical sensor based on the (PoANIS)ₓMoO₃ thin film of Example 3;
Fig. 19 is a graph plotting the differences between response values for various concentrations of formaldehyde and the response values for blank measurement, in the chemical sensor based on the (PoANIS)ₓMoO₃ thin film of Example 3; and
Fig. 20 is a graph plotting the differences between response values for various concentrations of acetaldehyde and the response values for blank measurement, in the chemical sensor based on the (PoANIS)ₓMoO₃ thin film of Example 3.

### DESCRIPTION OF THE PREFERRED EMBODIMENTS

The present invention is explained in detail next based on examples, although the invention is in no way meant to be limited to or by them.

### Example 1

### (1) Application of a lanthanum aluminate (LaAlO₃) buffer layer on a silicon (Si) substrate

A 85 mmol/L LaAlO₃ precursor solution was dripped on a 20 mm square Si substrate thermally oxidized film, followed by spin coating at 500 rpm for 10 seconds and then 3000 rpm for 30 seconds. Thereafter, the coated substrate was dried for about 30 minutes at 90°C, and was then sintered at 1100°C for 30 minutes. Through the above process there was coated a LaAlO₃ buffer layer, having a lattice constant close to that of molybdenum oxide, on a Si substrate provided with a thermally oxidized film.

### (2) Manufacture of a molybdenum oxide (MoO₃) thin film

A MoO₃ thin film was manufactured by CVD. Herein was used a Si substrate, having coated thereon a LaAlO₃ buffer layer, onto which there was vapor-deposited a gold comb electrode within a 10 mm square having an electrode width of 20 µm and an inter-electrode distance of 20 µm. This substrate was placed on a sample holder equipped with a heating heater. The substrate was moved from a source chamber into a sample chamber, the system interior was exchanged through flow of 50 mL/min of oxygen gas, then the sample holder was heated at 500°C, the sample chamber at 455°C, and the source chamber at 40°C.

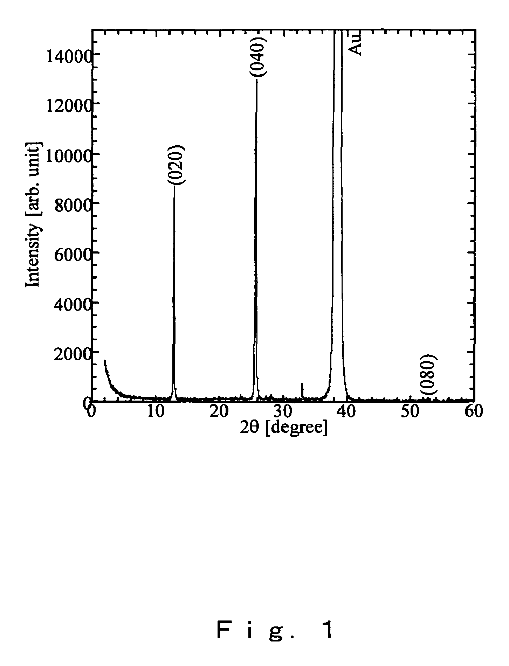

After temperature stabilization, a quartz glass boat filled with 0.35 g of molybdenum hexacarbonyl (Mo(CO)₆) was placed in the source chamber, then the pressure inside the system was reduced to 110 Pa using a vacuum pump. MoO₃ grew through vaporizing of Mo(CO)₆ under reduced pressure. After 15 minutes of film formation, the vacuum pump was stopped, and the entire system was reverted to atmospheric pressure, to discontinue film formation. Fig. 1 illustrates an X-ray diffraction pattern of the obtained MoO₃ thin film, measured with CuKα radiation. Excluding the peaks from the substrate and the peaks from the gold comb electrode, the observed diffraction peaks belonged to the layer-structure MoO₃ (0k0), wherein the MoO₃ thin film adopted a b-axis orientation vis-à-vis the substrate.

### (3) Manufacture of a [Na(H₂O)₂]ₓMoO₃ thin film

In a flask, 15 mL of distilled water were bubbled with nitrogen gas, under stirring, for 25 minutes, then therein was dissolved, as a buffering agent, disodium molybdenate (VI) dihydrate (Na₂MoO₄·2H₂O: 6 g). To this solution was then added sodium hyposulfite (Na₂S₂O₄ : 0.4 g). After dissolution of the latter, stirring and gas bubbling were stopped, then the MoO₃ thin film was immersed in the resulting solution for 20 seconds. The thin film turned from pale blue into blue as a result of the partial reduction of molybdenum.

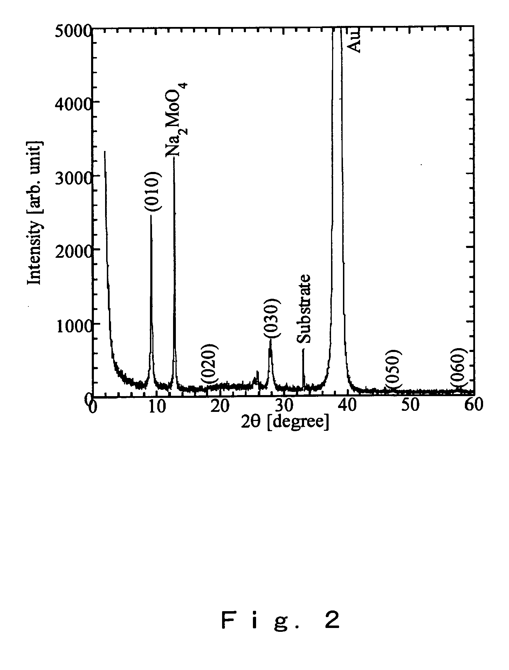

Thereafter, the thin film was washed with distilled water and was air-dried at 90°C for 30 minutes. Fig. 2 illustrates an X-ray diffraction pattern of the obtained thin film, measured with CuKα radiation. Excluding the peaks from the substrate, the peaks from the gold comb electrode, and the peaks from the buffering agent disodium molybdenate (VI) dihydrate, the observed diffraction peaks belonged to the layer-structure [Na(H₂O)₂]ₓMoO₃ (0k0). The interlayer distance increased by 2.7 Å to 9.6 Å, from a MoO₃ interlayer distance of 6.9 Å. This increase in the interlayer distance corresponds to the increase resulting from the intercalation of the hydrated sodium ions (Na(H₂O)₂) between layers, which gives rise to the formation of the [Na(H₂O)₂]ₓMoO₃ thin film.

### (4) Preparation of a polyaniline aqueous solution

In a flask, 1.4 mL of concentrated hydrochloric acid was stirred with 15 mL of distilled water, then 1 mL of the solution was transferred to another container. Aniline (1.5 mL, 16.5 mmol) was added then to the remaining 15.4 mL of hydrochloric acid aqueous solution, under stirring until homogenous solution, and under bubbling with nitrogen gas, to yield an aqueous solution of aniline hydrochloride. In the 1 mL of hydrochloric acid aqueous solution moved to a separate container there was dissolved ammonium persulfate ((NH₄)₂S₂O₈: 50 mg, 0.22 mmol) as a polymerization initiator. The ammonium persulfate aqueous solution was added to the aqueous solution of aniline hydrochloride, under stirring and continued nitrogen gas bubbling. After the addition, nitrogen gas bubbling continued, under stirring, for 30 minutes.

Bubbling and stirring were then stopped, and the solution was suction-filtered using a hydrophilic PTFE membrane filter having a diameter of 47 mm and a pore size of 0.5 µm.
Filtering was carried out herein to maximum suction, during 20 minutes, so as to obtain a filtrate of a solution containing insoluble polyaniline. The filtrate contained fiber-like insoluble polyaniline resulting from scraping of part of the filter owing to strong suction. Hence, the filtrate was suction-filtered again using a hydrophilic PTFE membrane filter having a diameter of 47 mm and a pore size of 0.5 µm, during 60 minutes, to yield polyaniline aqueous solution wholly free of insoluble polyaniline.

### (5) Preparation of a (PANI)ₓMoO₃ thin film

The above [Na(H₂O)₂]ₓMoO₃ thin film was soaked for 30 seconds in the obtained polyaniline aqueous solution, was washed with distilled water, was air-dried for 30 minutes, and was dried at 90°C to yield a (PANI)ₓMoO₃ thin film.

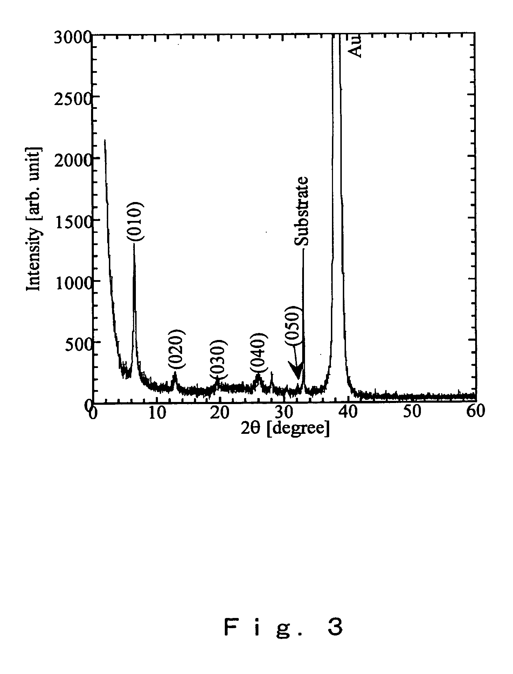

Fig. 3 illustrates an X-ray diffraction pattern of the obtained thin film, measured with CuKα radiation. Excluding the peaks from the substrate and the peaks from the gold comb electrode, the observed diffraction peaks belonged to layer-structure (PANI)ₓMoO₃ (0k0). The interlayer distance increased by 3.9 Å to 13.5 Å, from a [Na(H₂O)₂]ₓMoO₃ interlayer distance of 9.6 Å. This increase in the interlayer distance corresponds to the increase resulting from the intercalation of the PANI between layers, which gives rise to the formation of the (PANI)ₓMoO₃ thin film.

### (6) Evaluation of electric characteristic and sensor characteristic

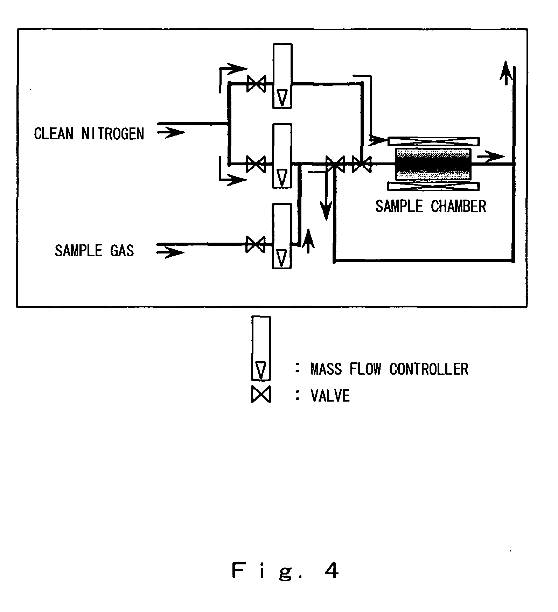

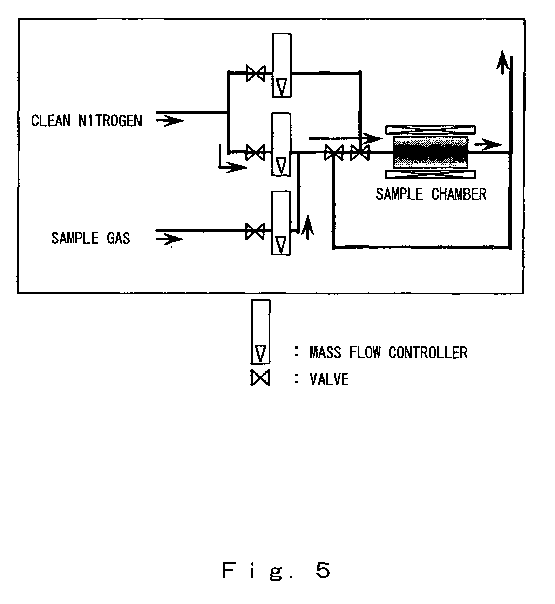

The sensor characteristic of the chemical sensor based on the (PANI)ₓMoO₃ thin film was evaluated based on variations of electric resistance values. The target VOC gas was formaldehyde or acetaldehyde. The measurements were carried out in an apparatus comprising a gas line, a valve, a mass flow controller, and a sample chamber, as illustrated in Figs. 4 and 5. A gold comb electrode was connected to a resistance measurement instrument inside the sample chamber, the interior of the sample chamber was heated at 30°C, and then measurements were carried out once the temperature had stabilized.

In an initial measurement, a nitrogen-based formaldehyde standard gas cylinder was connected to a sample gas line. As illustrated in Fig. 4, clean nitrogen was fed into the sample chamber, at a flow rate of 200 mL/min, and was also mixed, in parallel, with nitrogen-based formaldehyde standard gas, to a concentration of the latter of 25 ppb, and a total flow rate of 200 mL/min. This latter mixed gas was not fed into the sample chamber but was discharged out. After 30 minutes of gas flow in accordance with such a scheme, valves were switched to make the gases flow in accordance with the scheme illustrated in Fig. 5. Thenceforth, the 25 ppb formaldehyde gas was fed into the sample chamber at a flow rate of 200 mL/min. This gas flow scheme was maintained for 20 minutes. Thereafter, the valves were switched and, again, clean nitrogen was fed into the sample chamber at a flow rate of 200 mL/min, as illustrated in Fig. 4.

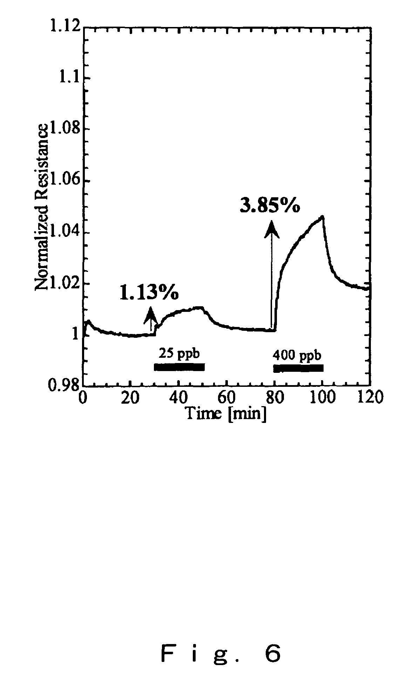

Now, the flow of nitrogen-based formaldehyde standard gas with clean nitrogen was regulated to yield a flow having a concentration of 400 ppb, to a total flow rate of 200 mL/min. This gas was not fed into the sample chamber but was discharged out. After 30 minutes of gas flow in accordance with such a scheme, the 400 ppb formaldehyde gas was fed again for 20 minutes into the sample chamber, in accordance with the flow scheme of Fig. 5. The flow scheme reverted then to that of Fig. 4, for another 20 minutes, after which the measurement ended. Fig. 6 illustrates the measurement results for sensor characteristic against 25 ppb and 400 ppb of formaldehyde. The percentages in Fig. 6 denote the amount of change in resistance value relative to the resistance value immediately before switching from clean nitrogen flowing into the sample chamber to the inflow of formaldehyde gas having the respective concentrations.

For a subsequent measurement, a nitrogen-based formaldehyde standard gas cylinder was connected to the sample gas line. As illustrated in Fig. 4, clean nitrogen was fed into the sample chamber, at a flow rate of 200 mL/min, and was also mixed, in parallel, with nitrogen-based formaldehyde standard gas, to a concentration of the latter of 50 ppb, and a total flow rate of 200 mL/min. This latter mixed gas was not fed into the sample chamber but was discharged out. After 30 minutes of gas flow in accordance with such a scheme, valves were switched to make the gases flow in accordance with the scheme illustrated in Fig. 5. Thenceforth, the 50 ppb formaldehyde gas was fed into the sample chamber at a flow rate of 200 mL/min. This gas flow scheme was maintained for 20 minutes.

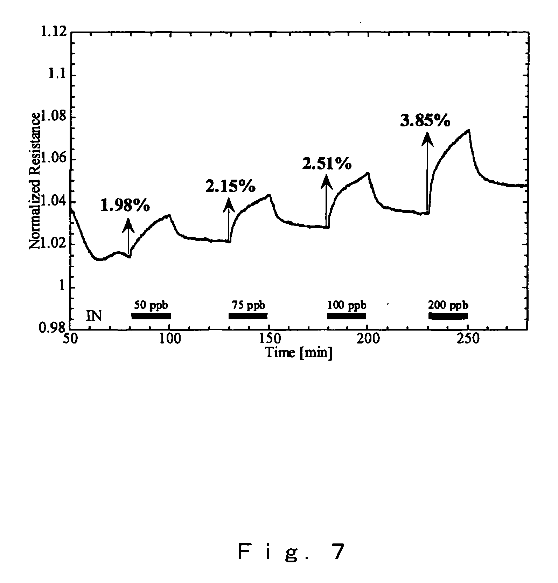

This operation was repeated for measuring resistance against concentrations of 75 ppb, 100 ppb and 200 ppb of formaldehyde gas. Lastly, the flow scheme was reverted thereafter to that of Fig. 4, for another 30 minutes, after which the measurement ended. Fig. 7 illustrates the measurement results for sensor characteristic against 50 ppb, 75 ppb, 100 ppb and 200 ppb of formaldehyde. The percentages in Fig. 7 denote the amount of change in resistance value relative to the resistance value immediately before switching from clean nitrogen flowing into the sample chamber to the inflow of formaldehyde gas having the respective concentrations. Figs. 6 and 7 indicate that the response of a chemical sensor using a (PANI)ₓMoO₃ thin film becomes stronger as the concentration increases from 25 ppb to 400 ppb.

For a subsequent measurement, a nitrogen-based acetaldehyde standard gas cylinder was connected to the sample gas line. As illustrated in Fig. 4, clean nitrogen was fed into the sample chamber, at a flow rate of 200 mL/min, and was also mixed, in parallel, with nitrogen-based acetaldehyde standard gas, to a concentration of the latter of 25 ppb, and a total flow rate of 200 mL/min. This latter mixed gas was not fed into the sample chamber but was discharged out. After 30 minutes of gas flow in accordance with such a scheme, valves were switched to make the gases flow in accordance with the scheme illustrated in Fig. 5. Thenceforth, the 25 ppb acetaldehyde gas was fed into the sample chamber at a flow rate of 200 mL/min. This gas flow scheme was maintained for 20 minutes.

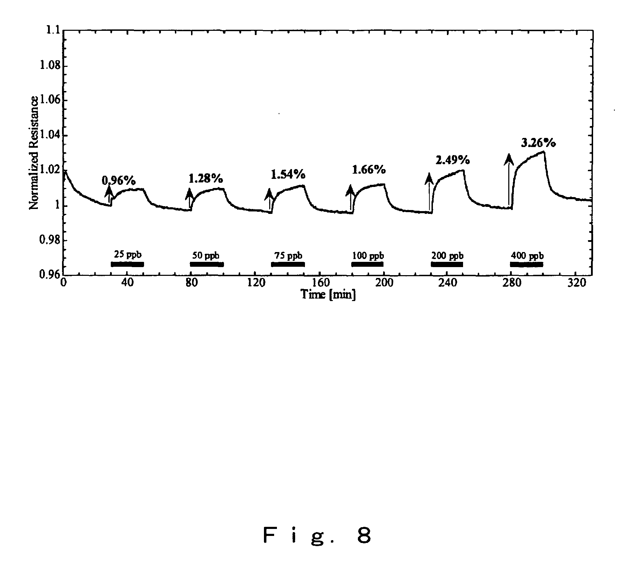

This operation was repeated for measuring resistance against concentrations of 50 ppb, 75 ppb, 100 ppb, 200 ppb and 400 ppb of acetaldehyde gas. Lastly, the flow scheme was reverted thereafter to that of Fig. 4, for another 30 minutes, after which the measurement ended. Fig. 8 illustrates the measurement results for sensor characteristic against 25 ppb, 50 ppb, 75 ppb, 100 ppb, 200 ppb and 400 ppb of acetaldehyde. The percentages in Fig. 8 denote the amount of change in resistance value relative to the resistance value immediately before switching from clean nitrogen flowing into the sample chamber to the inflow of acetaldehyde gas having the respective concentrations.

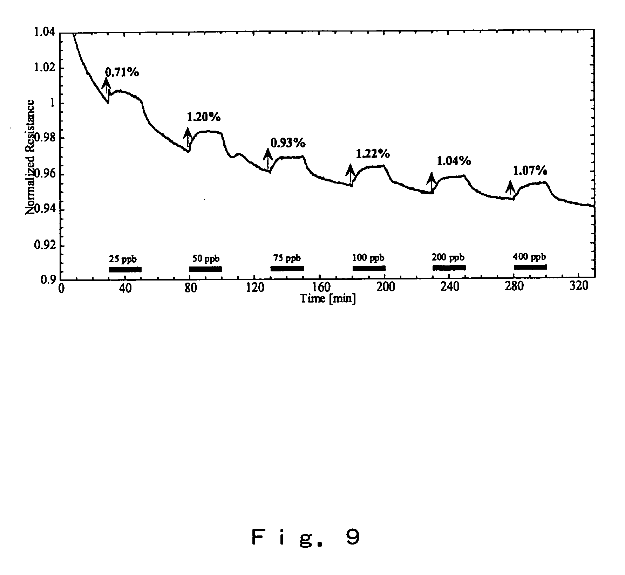

In a subsequent blank measurement the sample gas line was connected also in such a way so as to have clean nitrogen flowing therein. The blank measurement was carried out under exactly the same measurement conditions as in the measurement of response characteristics against 25 ppb, 50 ppb, 75 ppb, 100 ppb, 200 ppb and 400 ppb of formaldehyde or acetaldehyde. The response obtained as a result of these measurements varies depending on the measurement apparatus owing to small variations in temperature changes and/or pressure changes in the sample chamber, that result from the gases being introduced in the sample chamber via different gas lines through valve switching. The response value for aldehyde gases can be obtained accurately by factoring in the difference vis-à-vis the response value obtained in the blank measurement. Fig. 9 illustrates blank measurement results. The percentages in Fig. 9 denote the amount of change in resistance value relative to the resistance value immediately before switching between gases flowing into the sample chamber.

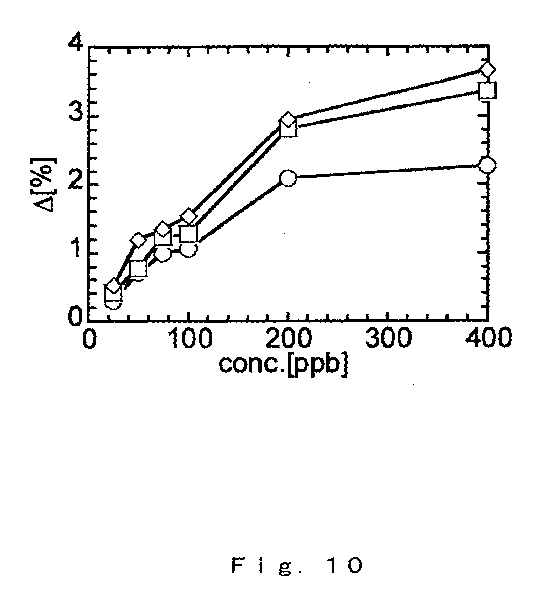

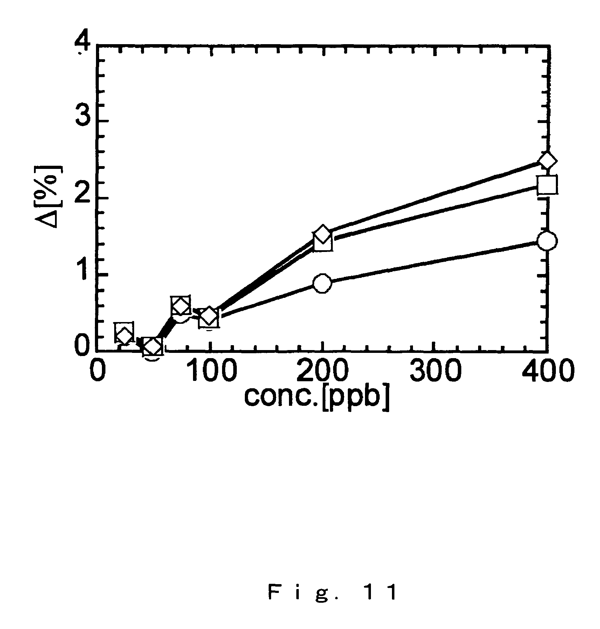

Fig. 10 is a graph in which there are plotted the differences between the response values for various formaldehyde concentrations, obtained in Figs. 6 and 7, and the response values for the blank measurement of Fig. 9. Fig. 11 is a graph in which there are plotted the differences between the response values versus various acetaldehyde concentrations, obtained in Fig. 8, and the response values for the blank measurement of Fig. 9. In Figs. 10 and 11, the results for three batches are plotted as (○ ), (□ ) and (◇ ), wherein the batches in Figs. 6, 7, 8 and 9 correspond to (□ ). The graphs indicate that a chemical sensor based on a (PANI)ₓMoO₃ thin film can sense ultra-low concentrations, from 25 ppb of formaldehyde, and from 75 ppb of acetaldehyde. Reproducibility was demonstrated based on the identical results obtained for the three batches.

### Example 2

In the present example there was manufactured a (PANI)ₓMoO₃ thin film by intercalating polyaniline between MoO₃ layers, according to a conventional procedure in which insoluble polyaniline remained dispersed in the intercalation polyaniline aqueous solution. In accordance with the process of Example 1, a LaAlO₃ buffer layer was coated on a Si substrate, a MoO₃ thin film was prepared thereon, and then a [Na(H₂O)₂]ₓMoO₃ thin film was manufactured. The (PANI)ₓMoO₃ thin film was manufactured in accordance with the process below.

In a flask, 15 mL of distilled water were bubbled with argon gas, under stirring, then thereto were added aniline (1.5 mL, 16.5 mmol) and concentrated hydrochloric acid (1.5 mL), to yield aniline hydrochloride. Thereto was added ammonium persulfate ((NH₄)₂S₂O₈: 50 mg, 0.22 mmol) as a polymerization initiator, while argon gas bubbling and stirring continued for 30 minutes. As a result, the aniline hydrochloride polymerized to yield not only polyaniline dissolved in the aqueous solution but also insoluble polyaniline having a high degree of polymerization. The [Na(H₂O)₂]ₓMoO₃ thin film was soaked for 30 seconds in the obtained polyaniline dispersion, was washed with distilled water, was air-dried for 30 minutes, and was dried at 90°C to yield a (PANI)ₓMoO₃ thin film.

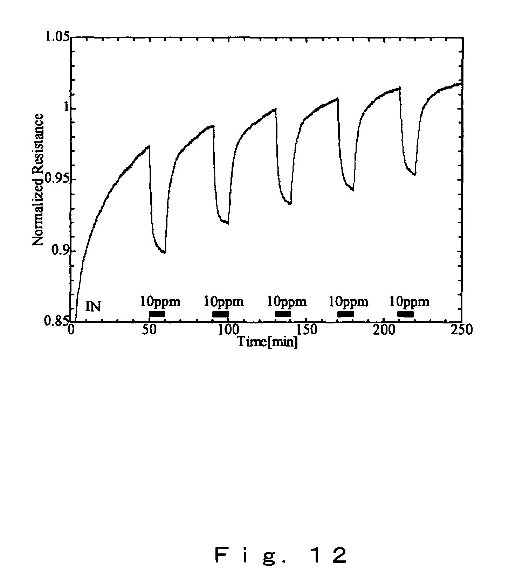

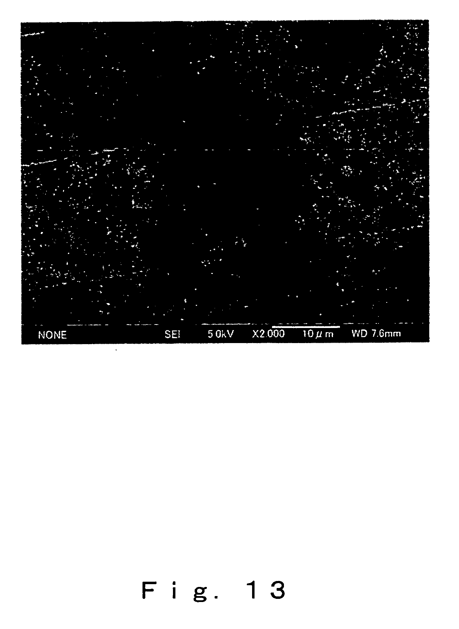

Fig. 12 illustrates response during alternate infusion of 10 ppm of acetaldehyde and clean nitrogen into the sample chamber of the apparatus for measuring sensor characteristics in Example 1. In a (PANI)ₓMoO₃ manufactured in accordance with the above operation, the resistance value decreases upon exposure to traces of an aldehyde gas, as illustrated in Fig. 12. Fig. 13 is a scanning electron micrograph of a (PANI)ₓMoO₃ thin film in which the resistance value decreases upon exposure to an aldehyde gas. Non-intercalated bulk polyaniline can be seen adhered across electrodes. Since the response of decreased resistance value versus an aldehyde gas is exhibited only by conductive polyaniline, such a response of decreased resistance value versus an aldehyde gas in the (PANI)ₓMoO₃ thin film manufactured in accordance with the procedure of the present example derives not from the (PANI)ₓMoO₃ hybrid, but from the polyaniline adhered to the surface of the (PANI)ₓMoO₃ thin film.

The (PANI)ₓMoO₃ thin film manufactured using the conventional method illustrated in Example 2 did not exhibit a response where the resistance value decreases for aldehyde gases such as those described above, and failed to detect an aldehyde gas in concentrations of several tens of ppb, in spite of the increased resistance value by the (PANI)ₓMoO₃ hybrid. Even if polyaniline not intercalated between MoO₃ layers did not become adhered across electrodes during the operation of polyaniline intercalation between MoO₃ layers in the conventional method illustrated in the present example, complete non-adhesion of polyaniline is not possible owing to the circumstances of the manufacturing procedure. The reason why the (PANI)ₓMoO₃ thin film manufactured in accordance with the conventional method illustrated in the present example cannot detect an aldehyde gas in concentrations of several tens of ppb is that the response of the MoO₃ hybrid is impaired by the diametrically opposite response from the small amounts of adhered polyaniline.

### Example 3

In the present example (PoANIS)ₓMoO₃ was manufactured through intercalation of poly(o-anisidine) between MoO₃ layers, and the electric and sensor characteristics of the (PoANIS)ₓMoO₃ were evaluated. According to the process of Example 1, a LaAlO₃ buffer layer was coated on a Si substrate, a MoO₃ thin film was formed, and then a [Na(H₂O)₂]ₓMoO₃ thin film was manufactured. The (PoANIS)ₓMoO₃ thin film was manufactured in accordance with the process below.

In a flask, 1.4 mL of concentrated hydrochloric acid was stirred with 15 mL of distilled water, then 1 mL of the solution was transferred to another container. Next, o-anisidine (1.86 mL, 16.5 mmol) was added to the remaining 15.4 mL of hydrochloric acid aqueous solution, under stirring until homogenous solution, and under bubbling with nitrogen gas, to yield an aqueous solution of o-anisidine hydrochloride. In the 1 mL of hydrochloric acid aqueous solution moved to a separate container there was dissolved ammonium persulfate ((NH₄)₂S₂O₈: 50 mg, 0.22 mmol) as a polymerization initiator. The ammonium persulfate aqueous solution was added to the aqueous solution of o-anisidine hydrochloride, under stirring and continued nitrogen gas bubbling. After the addition, nitrogen gas bubbling continued, under stirring, for 30 minutes.

Bubbling and stirring were then stopped, and the solution was suction-filtered using a hydrophilic PTFE membrane filter having a diameter of 47 mm and a pore size of 0.5 µm. Filtering was carried out herein to maximum suction, during 20 minutes, so as to obtain a filtrate of a solution containing insoluble poly(o-anisidine). Thereafter, the filtrate was suction-filtered again using a hydrophilic PTFE membrane filter having a diameter of 47 mm and a pore size of 0.5 µm, during 60 minutes, to yield a poly(o-anisidine) aqueous solution from which insoluble poly(o-anisidine) was wholly removed. The above [Na(H₂O)₂]ₓMoO₃ thin film was soaked for 30 seconds in the obtained poly(o-anisidine) aqueous solution, was washed with distilled water and was vacuum-dried for 1 hour to yield a (PoANIS)ₓMoO₃ thin film.

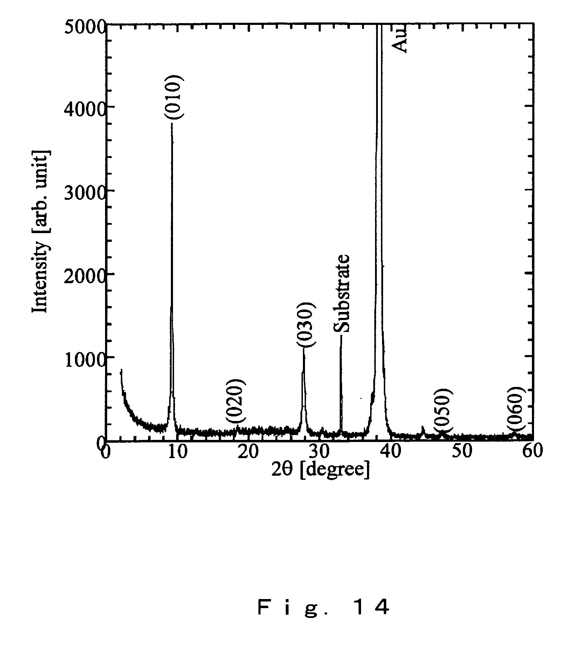

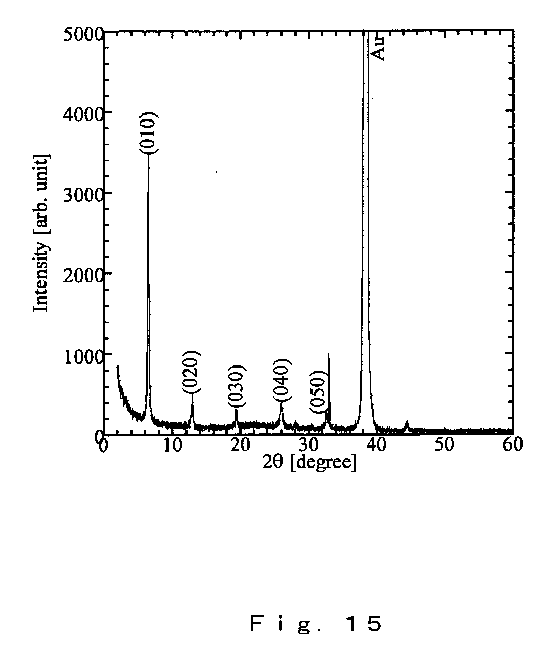

Fig. 14 illustrates an X-ray diffraction pattern of the [Na(H₂O)₂]ₓMoO₃ thin film immediately prior to soaking in the poly(o-anisidine) aqueous solution, measured with CuKα radiation. Fig. 15 illustrates an X-ray diffraction pattern of the obtained (PoANIS)ₓMoO₃ thin film, measured with CuKα radiation. Excluding the peaks from the substrate and the peaks from the gold comb electrode, the observed diffraction peaks belonged to layer-structure [Na(H₂O)₂]ₓMoO₃ or (PoANIS)ₓMoO₃ (0k0) . The interlayer distance increased by 4.1 Å to 13.7 Å, from a [Na(H₂O)₂]ₓMoO₃ interlayer distance of 9.6 Å. This increase in the interlayer distance corresponds to the increase resulting from the intercalation of the poly(o-anisidine) between layers, which gives rise to the formation of the (PoANIS)ₓMoO₃ thin film.

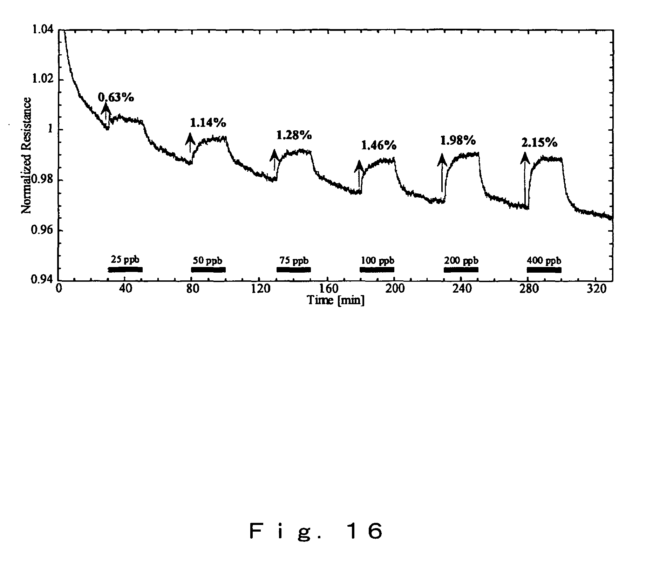

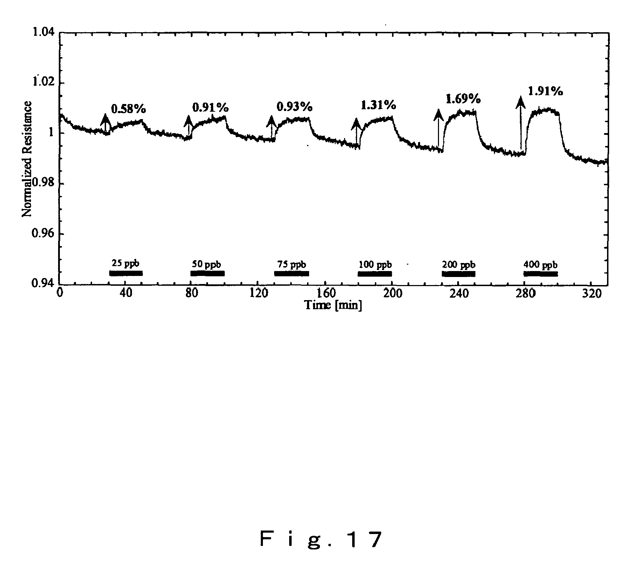

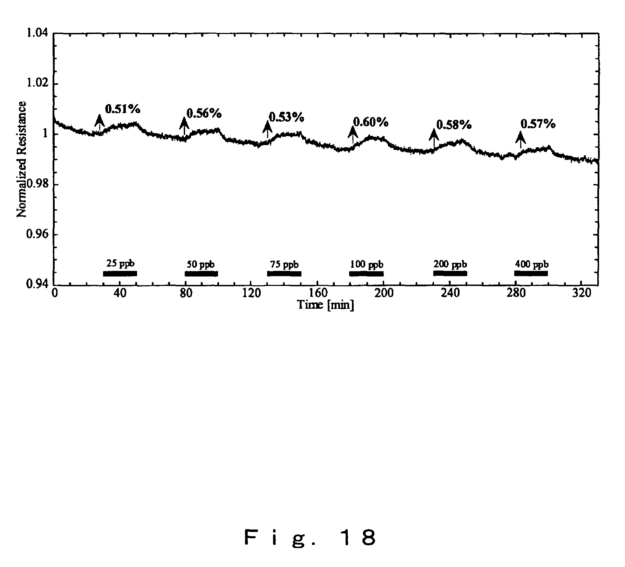

The sensor characteristic of a chemical sensor based on a (PoANIS)ₓMoO₃ thin film was measured using the apparatus illustrated in Example 1. The manufacturing process and the measurement conditions were the same as in Example 1. Fig. 16 and Fig. 17 illustrate the measurement results of sensor characteristic for 25 ppb, 50 ppb, 75 ppb, 100 ppb, 200 ppb and 400 ppb of formaldehyde and acetaldehyde, respectively. Fig. 18 illustrates the results for a blank measurement. The percentages in Figs. 16, 17 and 18 denote the amount of change in resistance value relative to the resistance value immediately before switching between gases flowing into the sample chamber.

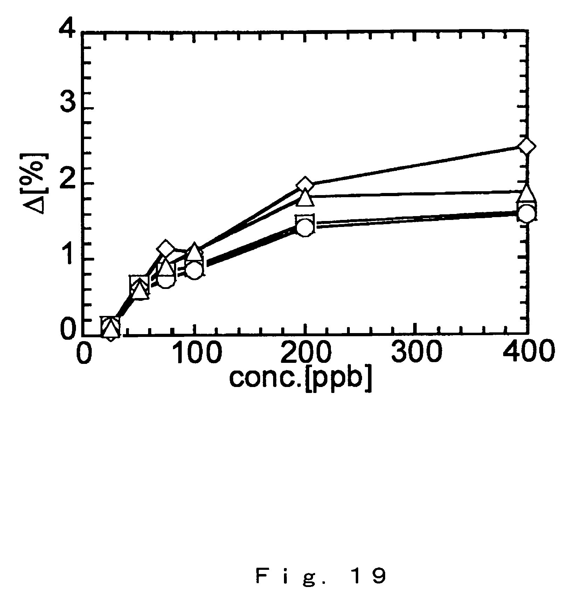

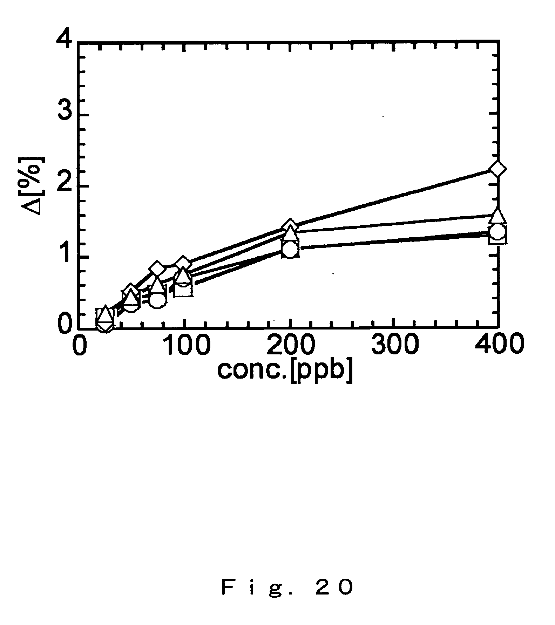

Fig. 19 is a graph in which there are plotted the differences between the response values to various formaldehyde concentrations, obtained in Fig. 16, and the response values for the blank measurement of Fig. 18. Fig. 20 is a graph in which there are plotted the differences between the response values to various acetaldehyde concentrations, obtained in Fig. 17, and the response values for the blank measurement of Fig. 18. In Figs. 19 and 20, the results for four batches are plotted as (○), (□ ) , (◇ ) and (Δ ), wherein the batches in Figs. 16, 17 and 18 correspond to (○ ). The graphs indicate that a chemical sensor based on a (PoANIS)ₓMoO₃ thin film can sense ultra-low concentrations, from 50 ppb of formaldehyde and 50 ppb of acetaldehyde. Reproducibility was demonstrated based on the identical results obtained for the four batches.

As described above, the present invention relates to a high sensitive gas sensor, and its manufacturing process, wherein the invention makes it possible to manufacture a gas sensor material, comprising an organic-inorganic hybrid material, that is capable of detecting aldehyde gases in concentrations of several tens of ppb. As a result, the chemical sensor of the present invention is capable of detecting by itself aldehyde gases at very low concentrations, of several tens of ppb, which are health-damaging concentrations. In addition to allowing constant monitoring of very low concentrations, the usefulness of the present invention lies also in providing a novel technology for widespread adoption of an inexpensive chemical sensor.

## Claims

1. A gas sensor material as a high sensitive gas sensor material for detecting an aldehyde gas in concentrations of several tens of ppb, comprising an organic-inorganic hybrid material in which a conductive organic polymer is intercalated between layers of an inorganic compound having a layer structure, in which the conductive organic polymer not intercalated between the layers of the inorganic compound is removed, and having sensitivity increased so as to detect an aldehyde gas in concentrations of several tens of ppb, on the basis of changes in resistance values.

2. The gas sensor material according to claim 1, wherein the shape of the organic-inorganic hybrid material, in which a conductive organic polymer is intercalated between layers of an inorganic compound having a layer structure, and from which a conductive organic polymer not intercalated between the layers of the inorganic compound is removed, is that of an oriented film.

3. The gas sensor material according to claim 1, wherein the inorganic compound having a layer structure is a compound having molybdenum oxide as a main component.

4. The gas sensor material according to claim 1, wherein the organic conductive polymer is a polymer having polyaniline as a main component.

5. The gas sensor material according to claim 1, wherein the organic conductive polymer is a polymer having a polyaniline derivative as a main component.

6. The gas sensor material according to claim 5, having as a main component a polyaniline derivative having an alkoxy group at the ortho position of the benzene ring.

7. A method for manufacturing a gas sensor material as a high sensitive gas sensor material for detecting an aldehyde gas in concentrations of several tens of ppb, comprising the steps of:
1) intercalating a conductive organic polymer between layers of an inorganic compound having a layer structure by using an aqueous solution from which an insoluble conductive organic polymer is removed, in a process of intercalating the conductive organic polymer between the layers of the inorganic compound;
2) producing thereby the gas sensor material having the organic-inorganic hybrid material in which the conductive organic polymer not intercalated between the layers of the inorganic compound is removed; and
3) increasing thereby sensitivity thereof so as to detect an aldehyde gas in concentrations of several tens of ppb, on the basis of changes in resistance values.

8. The process for manufacturing a gas sensor material according to claim 7, wherein the shape of the organic-inorganic hybrid material, in which a conductive organic polymer is intercalated between layers of an inorganic compound having a layer structure, and from which a conductive organic polymer not intercalated between the layers of the inorganic compound is removed, is that of an oriented film.

9. A chemical sensor member, comprising the gas sensor material defined in any one of claims 1 to 6 as a sensor element, and having a characteristic of detecting an aldehyde gas in concentrations of several tens of ppb.
